# EUROPEAN PATENT APPLICATION

(11) **EP 4 331 644 A1**
(43) Date of publication of application: **06.03.2024**
(21) Application number: 22193061.3
(22) Date of filing: 31.08.2022
(51) Int. Cl.: A61M 5/32

(54) **DEVICE FOR REMOVING AND REPLACING A NEEDLE SHEATH AND METHOD FOR DOING THE SAME**

(71) Applicant: B. Braun Medical Ltd., Sheffield Yorkshire S35 2PW (GB)
(72) Inventor: BRADLEY, Paul, Alnwick Northumberland, NE66 4TA (GB)
(74) Representative: Akers, Noel James

(57) **Abstract**

There is provided a device for removing and replacing a needle sheath of a needle device, the device comprising: a support; a needle sheath gripping member having an aperture therein, the aperture comprising: a needle sheath receiving portion having a first diameter for receiving a portion of the needle sheath therein; and a needle sheath gripping portion having a second diameter for gripping a portion of the needle sheath, wherein the second diameter is less than the first diameter, the sheath gripping portion comprising an edge for gripping the outer surface of the needle sheath. A method for removing a needle sheath from a needle device is also provided, the method comprising: inserting a portion of the needle sheath into a receiving portion of an aperture in a needle sheath gripping member; moving the needle sheath into a gripping portion of the aperture, whereby a portion of the outer surface of the needle sheath is gripped by opposing edge portions of the gripping portion of the aperture; and pulling the needle device away from the needle sheath gripping member.

## Description

The present invention relates to a device for removing and replacing a needle sheath covering the needle of a medical device, for example the sheath of a hypodermic syringe or an intravenous (IV) catheter or the like.

It is common practice to provide needles of medical devices, such as syringe needles, the needles of intravenous (IV) catheters and the like, with sheaths covering the needle tip and at least a portion of the shaft of the needle. The needle sheaths are typically made of plastic. The needle sheath is generally held in place over a needle by an interference fit with a portion of the medical device.

Before using the medical device, it is necessary for the medical practitioner to remove the needle sheath to reveal the needle and the sharpened needle tip. To expose the needle for use, the sheath must be pulled sharply to disengage from the medical device and be removed from covering the needle. When removing the needle sheath, for example by holding the device in one hand and pulling the needle sheath with the other hand, there is a risk of a needle stick injury. A greater risk of a needle stick injury arises if the medical practitioner attempts to replace the sheath over the tip of the needle. Replacing the sheath over the needle is advantageous, for example to avoid leaving the sharpened needle tip exposed with a consequent risk of a needle stick injury occurring. However, to replace the needle sheath, it is necessary to carefully guide the needle tip into the sheath and push the sheath to engage with the medical device.

There is therefore a need for an improved technique for safely removing a needle sheath from a medical device and, if required, to allow the sheath to be safely replaced over the needle. It would be particularly advantageous if the technique could significantly reduce or avoid the risk of a needle stick injury.

According to the present invention, there is provided a device for removing and replacing a needle sheath of a needle device, the device comprising:
a support;
a needle sheath gripping member having an aperture therein, the aperture comprising:
   a needle sheath receiving portion having a first diameter for receiving a portion of the needle sheath therein; and
   a needle sheath gripping portion having a second diameter for gripping a portion of the needle sheath, wherein the second diameter is less than the first diameter, the sheath gripping portion comprising an edge for gripping the outer surface of the needle sheath.

The device of the present invention allows the sheath of a medical needle, such as a hypodermic needle, IV catheter needle or the like, to be removed safely and replaced over the needle safely, reducing or eliminating the risk of a needle stick injury occurring. This is particularly the case when resheathing the needle, which generally requires the user to hold the sheath and guide the sharpened tip of the needle into the open end of the sheath, with the possible risk of a needle stick injury. By having the device retain the needle sheath, the task of introducing the needle into the sheath is made simpler, with a reduced risk of the sharpened needle tip contacting the user. In addition, the device allows the sheath to be removed from the needle, held by the device and replaced on the needle without the need for the user to touch the sheath. This in turn improves the aseptic technique of the user, in particular allowing the user to keep their fingers at a greater distance from the needle area.

The device comprises a support. The support provides support to the sheath gripping member, which is connected to the support.

In one embodiment, the support comprises a gripping portion that may be gripped by a user to hold the device and orient the needle sheath gripping portion for removing the sheath from covering the needle of a medical device or replacing the sheath over the needle.

In one embodiment, the support comprises a stand portion that may function as a stand, allowing the device to be placed on a surface with the needle sheath gripping portion oriented appropriately for the safe removal of the needle sheath from the medical device and the safe replacement of the sheath over the needle of the device.

In a preferred embodiment, the support comprises at least a portion that may be gripped by a user and functions as a stand.

In one preferred embodiment, the support comprises a first support member extending from a first side of the needle sheath gripping member and a second support member extending from a second side of the needle sheath gripping member, wherein the first side is preferably on an opposite side of the needle sheath gripping member to the second side.

The first and second support members may have any suitable form to allow the device to be gripped and/or supported. In one preferred embodiment, each of the first and second support members is a generally planar leg extending from a side of the needle sheath gripping member, each leg having a distal edge for contacting a surface on which the device is placed.

The first and second support members may have any suitable shape. In one preferred embodiment, each of the first and second support members is generally rectangular.

The device further comprises a needle sheath gripping member. The needle sheath gripping member may have any suitable form. In one embodiment, the needle sheath gripping member is generally planar. The needle sheath gripping member may have any suitable shape. In one preferred embodiment, the needle sheath gripping member is generally rectangular.

As noted above, the needle sheath gripping member is connected to the support. The needle sheath gripping member and the support may be formed as separate components, which are then connected together. More preferably, the needle sheath gripping member and the support are formed as a single component.

A preferred arrangement is to have the needle sheath gripping member and the support formed from a single plate, with the support having first and second support members, preferably opposing support members, which are formed by bending portions of the plate to the sides of the needle sheath gripping member, preferably on opposing sides of the needle sheath gripping member.

The needle sheath gripping member comprises an aperture therein. The apertures in the needle sheath gripping member comprises two portions: a needle sheath receiving portion; and a sheath gripping portion.

The needle sheath receiving portion has a first diameter. The diameter of the needle sheath receiving portion is of a size for receiving a portion of the needle sheath in the needle sheath receiving portion. The diameter of the needle sheath receiving portion is large enough to receive at least a portion of the needle sheath. In one preferred embodiment, the diameter of the needle sheath receiving portion is large enough that the needle sheath can be inserted into the needle sheath receiving portion and extend therethrough sufficiently for the distal end of the needle sheath to extend to or beyond the end of the support. In this way, when the device is placed on a surface, the needle sheath can be inserted into the needle sheath receiving portion of the aperture and extend to the surface. This advantageously simplifies the method of using the device for the medical practitioner. In particular, when replacing the needle sheath on the needle, the user can advance the tip of the needle into the open end of the needle sheath and push the needle device until the sheath is properly engaged. Movement of the sheath under the pushing action is prevented by the surface on which the device is resting.

The needle sheath receiving portion of the aperture may have any suitable diameter to allow at least a portion of the needle sheath to be received therein and extend therethrough. The needle sheath receiving portion may have a diameter of at least 4mm, preferably at least 4.5mm, more preferably at least 5mm, still more preferably at least 5.5mm, more preferably still at least 6mm, especially at least 6.5mm, more especially at least 7mm. The needle sheath receiving portion may have a diameter of up to 11mm, preferably up to 10.5mm, more preferably up to 10mm, still more preferably up to 9.5mm, more preferably still up to 9mm, especially up to 8.5mm, more especially up to 8mm. The needle sheath receiving portion may have a diameter of 4 to 11mm, preferably from 4.5 to 10.5mm, more preferably from 5 to 10mm, still more preferably from 5.5 to 9.5mm, more preferably still from 6 to 9mm, especially from 6.5 to 8.5mm, more especially from 7 to 8mm. In one preferred embodiment, the needle sheath receiving portion has a diameter of from 7.2 to 7.9mm, preferably from 7.3 to 7.8mm, more preferably from 7.4 to 7.75mm, still more preferably from 7.5 to 7.7mm. A preferred diameter for the needle sheath receiving portion of the aperture is about 7.65mm.

The aperture also comprises a sheath gripping portion. The sheath gripping portion has a second diameter. The second diameter is of a size to allow the sheath gripping portion to grip a portion of the needle sheath. The second diameter is less than the first diameter.

The needle sheath gripping portion of the aperture may have any suitable diameter to allow at a portion of the needle sheath to be griped by the edges of the needle sheath gripping portion. The needle sheath gripping portion may have a diameter of at least 2mm, preferably at least 2.5mm, more preferably at least 3mm, still more preferably at least 3.5mm, more preferably still at least 4mm, especially at least 4.5mm, more especially at least 5mm. The needle sheath gripping portion may have a diameter of up to 9mm, preferably up to 8.5mm, more preferably up to 8mm, still more preferably up to 7.5mm, more preferably still up to 7mm, especially up to 6.5mm, more especially up to 6mm. The needle sheath gripping portion may have a diameter of 2 to 9mm, preferably from 2.5 to 8.5mm, more preferably from 3 to 8mm, still more preferably from 3.5 to 7.5mm, more preferably still from 4 to 7mm, especially from 4.5 to 6.5mm, more especially from 5 to 6mm. In one preferred embodiment, the needle sheath gripping portion has a diameter of from 5.1 to 5.9mm, preferably from 5.2 to 5.8mm, more preferably from 5.3 to 5.7mm, still more preferably from 5.4 to 5.6mm. A preferred diameter for the needle sheath receiving portion of the aperture is about 5.5mm.

The needle sheath gripping portion of the aperture functions to grip a portion of the needle sheath, holding the needle sheath sufficiently firmly that the needle device may be pulled away from the needle sheath gripping member, disengaging the needle sheath from the needle device and allowing the needle to be removed from the sheath. The needle sheath is gripped by the edge of the needle sheath gripping portion, preferably opposing edge portions of the needle sheath gripping portion of the aperture.

The needle sheath gripping member may have any suitable thickness in the region of the needle sheath gripping portion of the aperture for allowing the edge of the needle sheath gripping portion to grip the needle sheath. Preferably, the needle sheath gripping member has a thickness in the region of the needle sheath gripping portion of the aperture of from 0.5mm, more preferably from 0.7mm, still more preferably from 0.9mm, more preferably still from 1mm, more preferably still from 1.2mm, especially from 1.3mm, more especially from 1.4mm. Preferably, the needle sheath gripping member has a thickness in the region of the needle sheath gripping portion of the aperture of up to 2.2mm, more preferably up to 2.1mm, still more preferably up to 2mm, more preferably still up to 1.9mm, more preferably still up to 1.8mm, especially up to 1.7mm, more especially up to 1.6mm. Preferably, the needle sheath gripping member has a thickness in the region of the needle sheath gripping portion of the aperture of from 0.5 to 2.2mm, more preferably from 0.7 to 2.1mm, still more preferably from 0.9 to 2mm, more preferably still from 1 to 1.9mm, more preferably still from 1.2 to 1.8mm, especially from 1.3 to 1.7mm, more especially from 1.4 to 1.6mm. A preferred thickness for the region of the needle sheath gripping member surrounding the needle sheath gripping portion of the aperture is about 1.5mm.

The edge of the needle sheath gripping portion of the aperture preferably comprises one or more chamfers to define a sharp edge for gripping the outer surface of the needle sheath. The edge of the needle sheath gripping portion may have a single chamfer portion that grips one side of the needle sheath. More preferably, the edge of the needle sheath gripping portion of the aperture comprises two opposing chamfer portions, each defining a sharp edge for gripping opposing portions of the outer surface of the needle sheath. In use the needle sheath is held between and gripped by the opposing chamfer portions. Preferably, the two chamfers are equal and arranged symmetrically about the centre of the edge. In one preferred embodiment, the needle sheath gripping portion is provided with a chamfer along the entire length of its edge.

The or each chamfer on the edge of the needle sheath gripping portion of the aperture may extend at any suitable angle to the surface of the needle sheath gripping member. The angle may be from 20°, preferably from 22.5°, more preferably from 25°, still more preferably from 27.5°. The angle may be up to 40°, preferably up to 37.5°, more preferably up to 35°, still more preferably up to 32.5°. The angle may be from 20 to 40°, preferably from 22.5 to 37.5°, more preferably from 25 to 35°, still more preferably from 27.5 to 32.5°. An angle of from 28 to 32° is particularly preferred, especially from 29 to 31°, more especially about 30°.

The or each chamfer is preferably provided on at least two opposing portions of the edge of the needle sheath gripping portion of the aperture, more preferably along substantially the entire length of the edge of the needle sheath gripping portion.

In one embodiment, at least a major portion or all of the edge of the needle sheath receiving portion of the aperture is not provided with a chamfer. In one preferred embodiment, part or all of the edge of the needle sheath receiving portion of the aperture is provided with a chamfer. For example, the portions of the edge of the needle sheath receiving portion of the aperture adjacent or closest to the needle sheath gripping portion may be provided with a chamfer. In one embodiment, the entire needle sheath receiving portion of the aperture is provided with a chamfer. Features of the chamfer are as hereinbefore described.

The aperture in the needle sheath gripping member may have any suitable shape to provide the needle sheath receiving portion and the needle sheath gripping portion. Moving from the needle sheath receiving portion to the needle sheath gripping portion, the diameter of the aperture may reduce in a continuous manner or in a stepwise manner. In one embodiment, the aperture in the needle sheath gripping member comprises a transition portion extending between the needle sheath receiving portion and the needle sheath gripping portion. In use, the needle sheath is inserted into the needle sheath receiving portion of the aperture and move along the transition portion into the needle sheath gripping portion, where the needle sheath is gripped by the edges of the needle sheath gripping portion and held. The transition portion may have any suitable form. In one embodiment, the transition portion is tapered, having a diameter that decreases in the direction from the needle sheath receiving portion to the needle sheath gripping portion. In one embodiment, the transition portion is a tapered slot.

In one embodiment, at least a major portion or all of the edge of the transition portion of the aperture is not provided with a chamfer. In one preferred embodiment, part or all of the edge of the transition portion of the aperture is provided with a chamfer. For example, the portions of the edge of the transition portion of the aperture adjacent or closest to the needle sheath gripping portion may be provided with a chamfer. In one embodiment, the entire transition portion of the aperture is provided with a chamfer. Features of the chamfer are as hereinbefore described.

The needle sheath receiving portion and/or the needle sheath gripping portion preferably have at least a portion that is a segment of a circle.

The aperture in the needle sheath gripping member may comprise a single needle sheath gripping portion. Alternatively, the aperture may comprise a plurality of needle sheath gripping portions, preferably two needle sheath gripping portions, more preferably arranged on opposite sides of the needle sheath receiving portion of the aperture. The form of the two or more needle sheath gripping portions may be the same or different, preferably the same.

Examples of suitable shapes for the aperture in the needle sheath gripping member include a teardrop shape, generally triangular, and diamond shaped.

The device of the present invention may be formed from any suitable material. For example, the device may be formed from a medical grade plastic. Suitable medical grade plastics are known in the art. The plastic should preferably have a hardness greater than the hardness of the material of the needle sheath. More preferably, the device is formed from metal, with a preference for steel, in particular stainless steel.

In use, the needle sheath to be removed from the needle device is inserted into the needle sheath receiving portion of the aperture in the needle sheath gripping member. The needle sheath is then pushed into the needle sheath gripping portion of the aperture until the edges of the needle sheath gripping portion grip the outer surface of the needle sheath. The needle device may then be pulled away from the device, removing the needle sheath and exposing the needle. The needle sheath preferably remains gripped in the needle sheath gripping portion of the aperture, allowing the needle to be reinserted into the sheath and the sheath reengaged with the needle device. The needle sheath is moved from the needle sheath gripping portion to the needle sheath receiving portion of the aperture, from where it can be removed.

Preferably, the device is placed on a surface, such as a table, desk or bench, and held on the surface. In use, the needle sheath is preferably inserted into the aperture in the needle sheath gripping member until the distal end of the needle sheath is in contact with the surface. This provides a particularly stable method of use, with reduced risks for a needle stick injury.

In a further aspect, the present invention provides a method for removing a needle sheath from a needle device, the method comprising:
inserting a portion of the needle sheath into a receiving portion of an aperture in a needle sheath gripping member;
moving the needle sheath into a gripping portion of the aperture, whereby a portion of the outer surface of the needle sheath is gripped by opposing edge portions of the gripping portion of the aperture; and
pulling the needle device away from the needle sheath gripping member.

The method may further comprise the further steps of:
inserting the needle of the needle device into the needle sheath and reengaging the needle sheath with the needle device;
moving the needle sheath from the gripping portion to the receiving portion of the aperture; and
pulling the needle device away from the needle sheath gripping member.

As discussed above, preferably the needle sheath gripping member is held on a surface.

Embodiments of the device of the present invention will now be described, by way of example only, having reference to the accompanying drawings, in which:
Figure 1 is a perspective view of one embodiment of the device of the present invention;
Figure 2 is a plan view of the device of Figure 1 in the direction of arrow A;
Figure 2a is an enlarged view of a portion of the device of Figure 2 showing the aperture in the needle sheath gripping member;
Figure 2b is an enlarged view of a portion of the device of Figure 2 showing an alternative embodiment of the aperture in the needle sheath gripping member;
Figure 3a is a cross-sectional view of a portion including the aperture of the device of Figure 2a along the line IIIa - IIIa in Figure 2a;
Figure 3b is a cross-sectional view of a portion including the aperture of the device of Figure 2b along the line IIIb - IIIb in Figure 2b;
Figure 4 is a plan view of a second embodiment of the device of the present invention;
Figure 5 is a plan view of a third embodiment of the device of the present invention;
Figure 6 is a plan view of a fourth embodiment of the device of the present invention;
Figure 7 is a plan view of a fifth embodiment of the device of the present invention;
Figure 8 is a plan view of a sixth embodiment of the device of the present invention; and
Figure 9 is a diagram showing the needle sheath of a needle device held in the device of Figure 1.

The terms 'upper' and 'lower' are used herein to describe the arrangement of components and parts of the device as they are oriented during normal use of the device and as shown in figures.

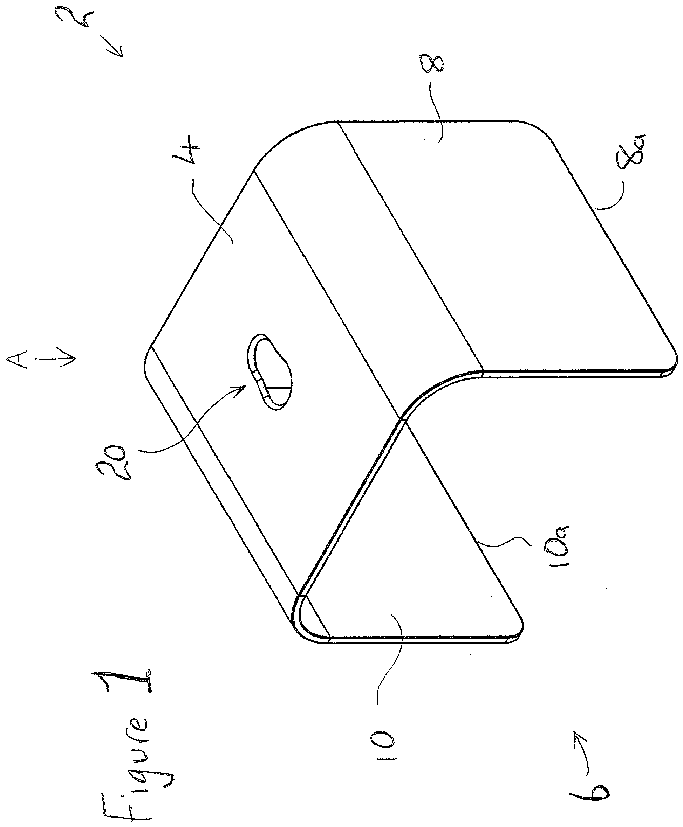

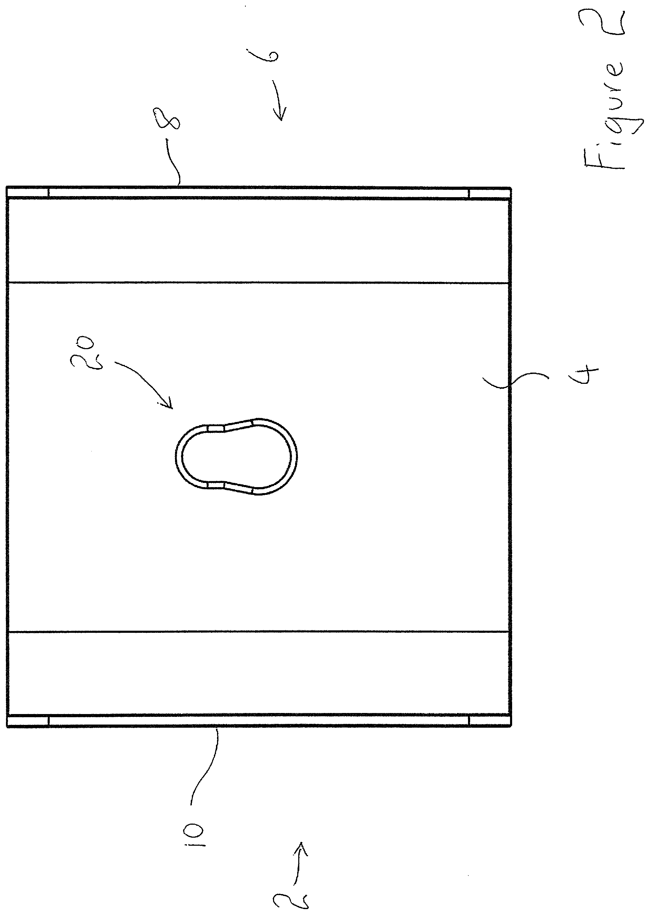

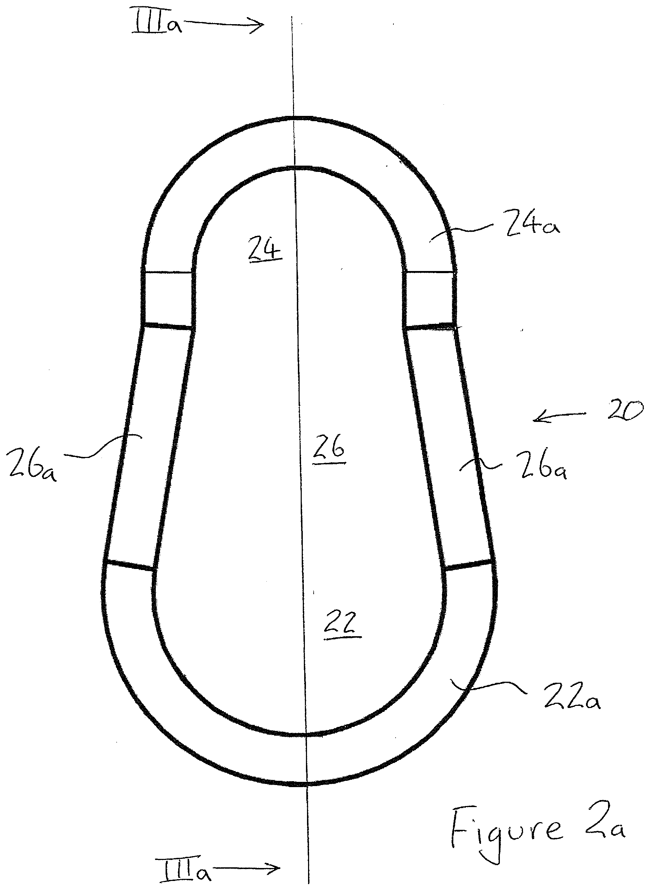

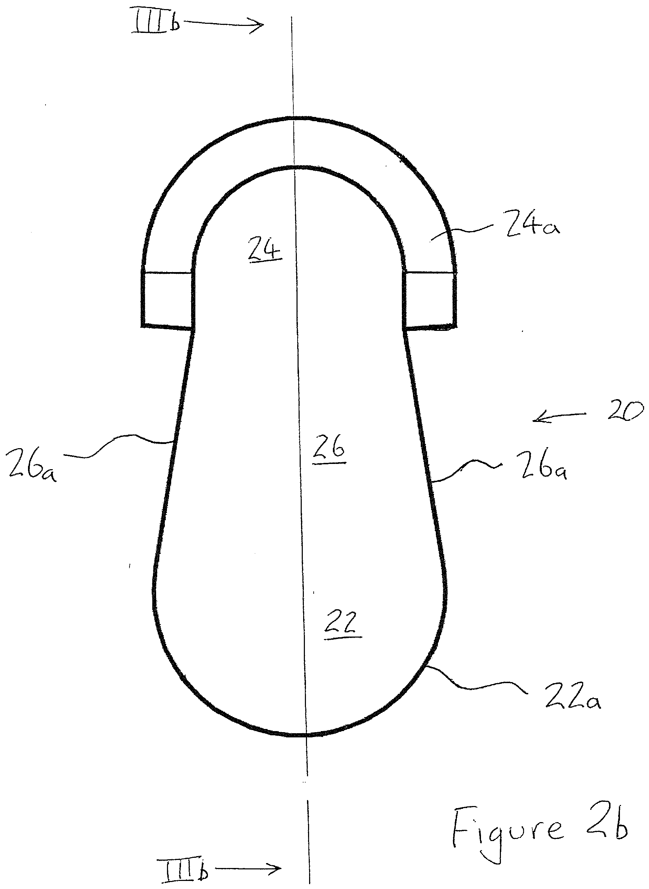

Turning to Figure 1, there is shown a perspective view of one embodiment of the device of the present invention. The device is shown in plan view in Figure 2. The device, generally indicated as 2, comprises a planar, generally rectangular needle sheath gripping member 4. The device 2 further comprises a support 6 comprising a first support member 8 extending from one side of the needle sheath gripping member 4 and a second support member 10 extending from the opposite side of the needle sheath gripping member 4. Each of the first and second support members 8, 10 extend generally perpendicular to the needle sheath gripping member and are planar, generally rectangular in form. Each of the first and second support members 8, 10 has a lower edge 8a, 10a. In use, the device 2 is oriented as shown in Figure 1 with the lower edges 8a, 10a of the first and second support members 8, 10 resting on a surface.

In the embodiment shown in Figure 1, the device 2 is formed as a single component from stainless steel, in particular from an elongate plate of stainless steel with the first and second support members 8, 10 formed by bending the end portions of the elongate plate.

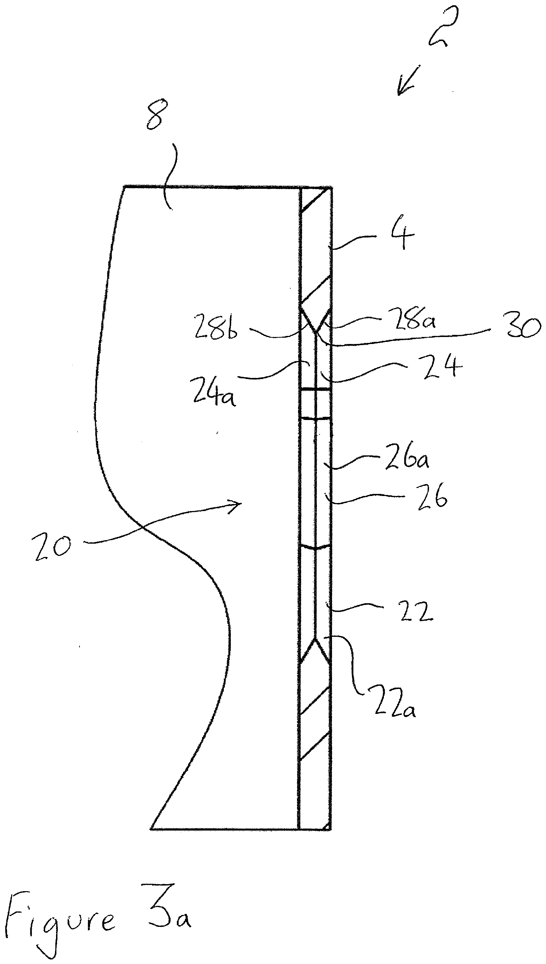

The needle sheath gripping member 4 comprises an aperture 20 disposed centrally therein, as shown more clearly in Figure 2. The aperture 20 is shown in more detail in Figure 2a. The aperture is shown in cross-section in Figure 3a.

The aperture 20 comprises a needle sheath receiving portion 22 and a needle sheath gripping portion 24.

The needle sheath receiving portion 22 of the aperture 20 is generally semicircular and lies at one end of the aperture 20. The needle sheath gripping portion 24 is generally U-shaped and lies at the opposite end of the aperture 20 to the needle sheath receiving portion 22. A transition portion of the aperture 20, in the form of a tapered slot 26 extends between the needle sheath receiving portion 22 and the needle sheath gripping portion 24.

In the embodiment shown in Figure 2a, the needle sheath receiving portion 22 has a diameter of from 7.6 to 7.7mm. The needle sheath gripping portion 24 has a smaller diameter of from 5.45 to 5.55mm.

The needle sheath gripping portion 24 of the aperture 20 is provided with a chamfered edge 24a. In particular, the upper and lower edges of the needle sheath gripping portion 24 are provided with a chamfer 28a, 28b. The chamfers 28a, 28b are symmetrical about the centre line of the edge and extend at an angle of 30° to the respective major surfaces of the needle sheath gripping member 4 to form a sharp edge 30, as shown more clearly in Figure 3a. In use, the sharp edge 30 grips opposing portions of the outer surface of the needle sheath.

The needle sheath receiving portion 22 has an edge 22a. The edge 22a may be a plain edge or may be chamfered. In one embodiment, the edge 22a has a chamfer at least on the portions adjacent the slot 26. In another embodiment, the edge 22a is provided with a chamfer along its entire length, as shown in Figure 2a. The form of the chamfer on the edge 22a is the same as that of the chamfer applied to the edge 24a of the needle sheath receiving portion 24, shown in Figure 3a and described above.

The slot 26 has an edge 26a on each side. The edges 26a may each be a plain edge or may be chamfered. In one embodiment, each edge 26a of the slot 26 has a chamfer at least on the portions adjacent the needle sheath gripping portion 24. In another embodiment, the edges 26a of the slot 26 are each provided with a chamfer along the entire length of the edge, as shown in Figure 2a. The form of the chamfer on the edges 26a is the same as the form of the chamfer applied to the edge 24a of the needle sheath receiving portion 24, shown in Figure 3a and described above.

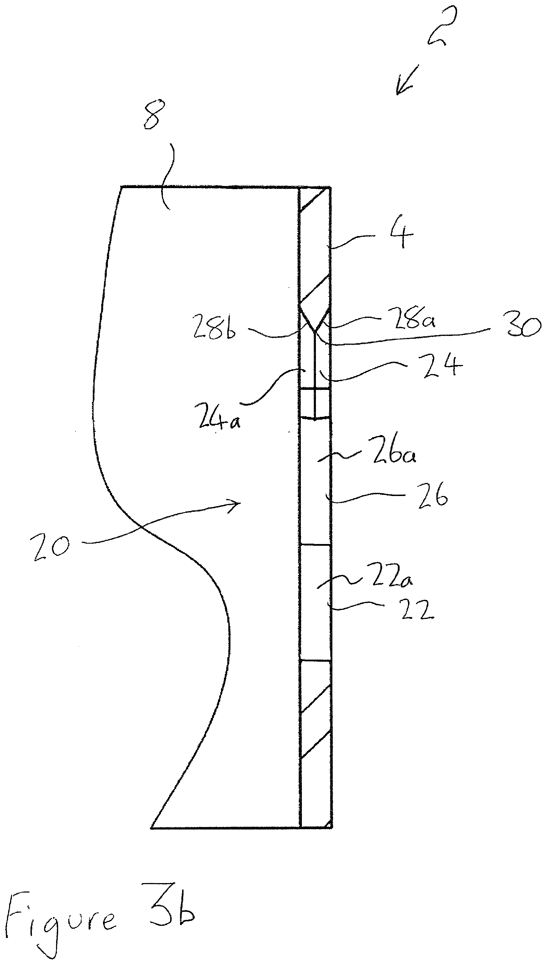

As noted above, in one embodiment, the edges 22a and 26a of the needle sheath receiving portion 22 and the slot 26 are not provided with chamfers. This embodiment is shown in Figure 2b. A cross-section of the aperture of this embodiment is shown in Figure 3b.

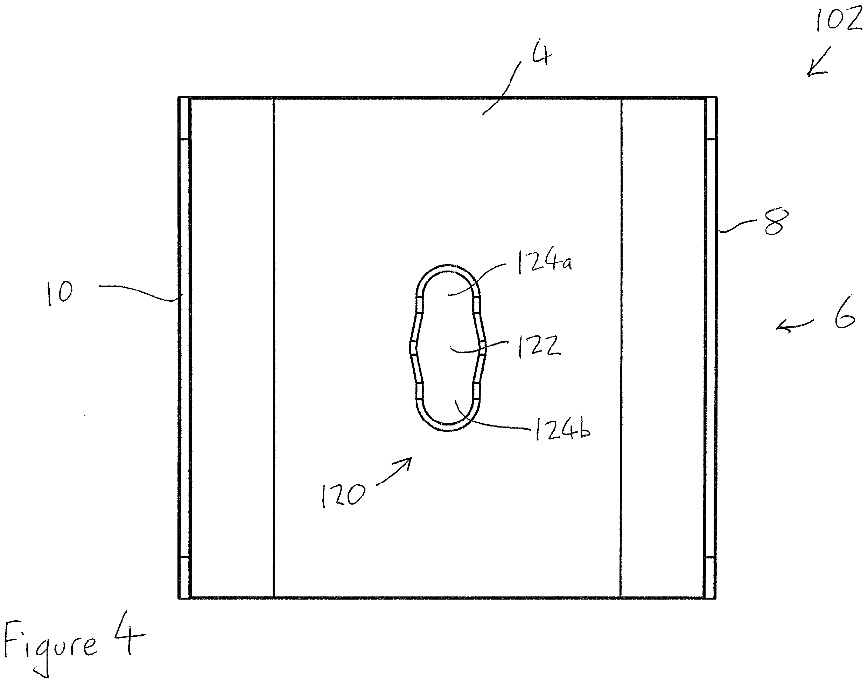

Turning to Figure 4, there is shown a plan view of a device according to a second embodiment of the present invention. The device, generally indicated as 102, has the same general form as the device of Figures 1 to 3 and the components common to the embodiments have been indicated using the same reference numerals and are as described above.

The embodiment of Figure 4 differs from the embodiment of Figure 1 with respect to the shape of the aperture 120 in the needle sheath gripping member 4. As can be seen in Figure 4, the aperture 120 is an elongate opening having a needle sheath receiving portion 122 disposed centrally in the aperture 120. First and second needle sheath gripping portions 124a and 124b are disposed at opposite ends of the aperture 120. Each of the needle sheath gripping portions 124a, 124b has the general form, including the chamfers and sharp edge, as the gripping portion 24 of the embodiment of Figures 1 to 3 and described hereinbefore.

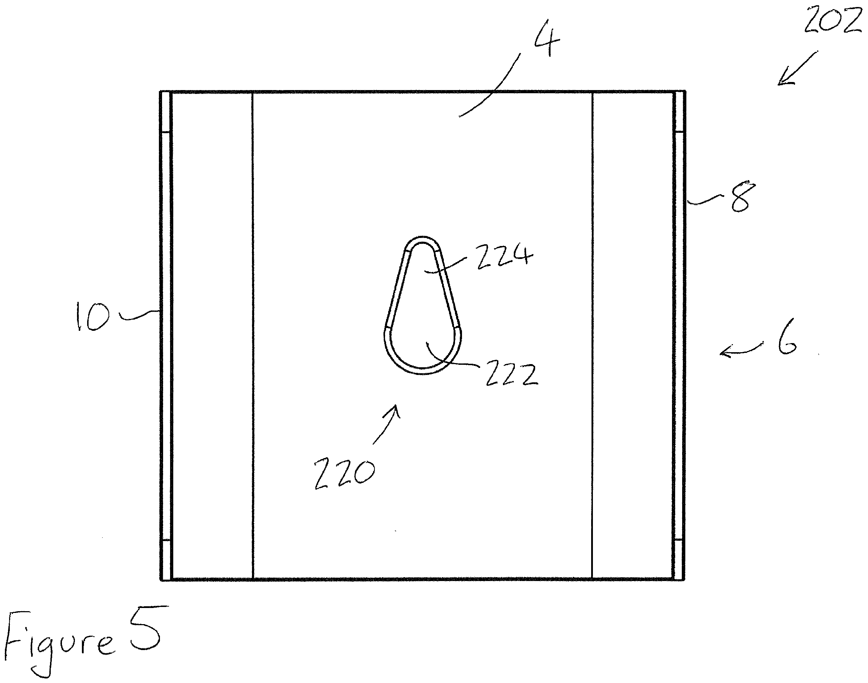

Turning to Figure 5, there is shown a plan view of a device according to a third embodiment of the present invention. The device, generally indicated as 202, has the same general form as the device of Figures 1 to 3 and the components common to the embodiments have been indicated using the same reference numerals and are as described above.

The embodiment of Figure 5 differs from the embodiment of Figure 1 with respect to the shape of the aperture 220 in the needle sheath gripping member 4. As can be seen in Figure 5, the aperture 220 is an opening in the shape of a teardrop having a needle sheath receiving portion 222 disposed at one end of the aperture 220 and a needle sheath gripping portion 224 disposed at the opposite end of the aperture 220. The needle sheath gripping portion 224 has the same general form of the chamfers and sharp edge as the gripping portion 24 of the embodiment of Figures 1 to 3 and described hereinbefore.

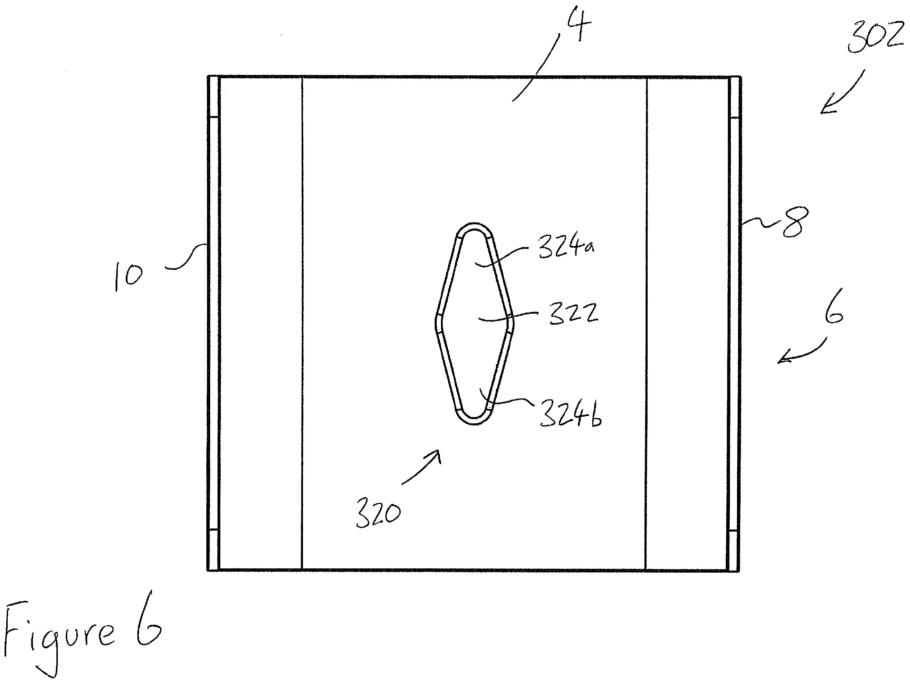

Turning to Figure 6, there is shown a plan view of a device according to a fourth embodiment of the present invention. The device, generally indicated as 302, has the same general form as the device of Figures 1 to 3 and the components common to the embodiments have been indicated using the same reference numerals and are as described above.

The embodiment of Figure 6 differs from the embodiment of Figure 1 with respect to the shape of the aperture 320 in the needle sheath gripping member 4. As can be seen in Figure 6, the aperture 320 is an elongate generally diamond shape having a needle sheath receiving portion 322 disposed centrally in the aperture 320. First and second needle sheath gripping portions 324a and 324b are disposed at opposite ends of the aperture 320. Each of the needle sheath gripping portions 324a, 324b has the same general form of chamfers and sharp edge as the gripping portion 24 of the embodiment of Figures 1 to 3 and described hereinbefore.

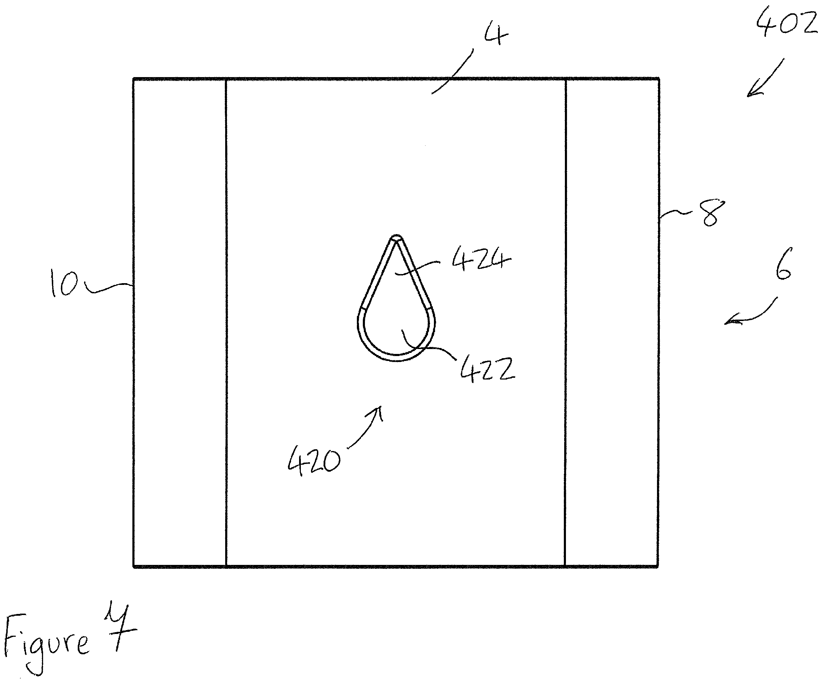

Turning to Figure 7, there is shown a plan view of a device according to a fifth embodiment of the present invention. The device, generally indicated as 402, has the same general form as the device of Figures 1 to 3 and the components common to the embodiments have been indicated using the same reference numerals and are as described above.

The embodiment of Figure 7 differs from the embodiment of Figure 1 with respect to the shape of the aperture 420 in the needle sheath gripping member 4. As can be seen in Figure 7, the aperture 420 is an opening in the shape of a teardrop having a needle sheath receiving portion 422 disposed at one end of the aperture 420 and a needle sheath gripping portion 424 disposed at the opposite end of the aperture 420. The needle sheath gripping portion 424 of the aperture tapers to a point, compared with the rounded gripping portion 224 of the embodiment of Figure 5. The needle sheath gripping portion 424 has the same general form of the chamfers and sharp edge as the gripping portion 24 of the embodiment of Figures 1 to 3 and described hereinbefore.

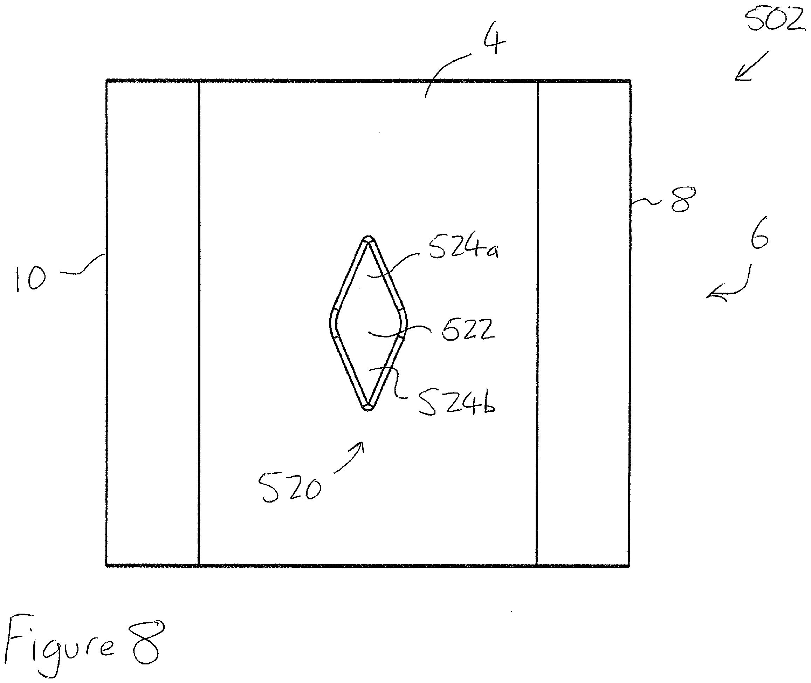

Turning to Figure 8, there is shown a plan view of a device according to a fifth embodiment of the present invention. The device, generally indicated as 502, has the same general form as the device of Figures 1 to 3 and the components common to the embodiments have been indicated using the same reference numerals and are as described above.

The embodiment of Figure 8 differs from the embodiment of Figure 1 with respect to the shape of the aperture 520 in the needle sheath gripping member 4. As can be seen in Figure 8, the aperture 520 is an opening in the shape of an elongate diamond having a needle sheath receiving portion 522 disposed centrally in the aperture 520. First and second needle sheath gripping portions 524a and 524b are disposed at opposite ends of the aperture 520. Each of the gripping portions 524a and 524b tapers to a point, compared with the rounded gripping portions 324a and 324b of the embodiment of Figure 6. Each of the needle sheath gripping portions 524a, 524b has the same general form of chamfers and sharp edge as the gripping portion 24 of the embodiment of Figures 1 to 3 and described hereinbefore.

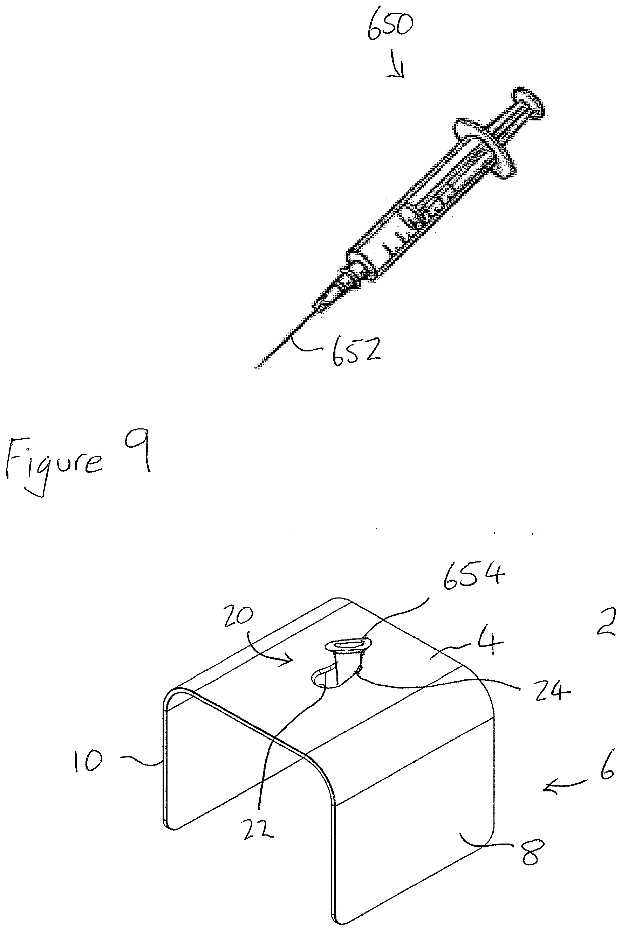

Turning to Figure 9, there is shown a device of the embodiment of Figure 1 in use with a hypodermic syringe. The hypodermic syringe 650 has a needle 652 and a needle sheath 654. The needle sheath 652 in place on the syringe 650 has been inserted into the needle sheath receiving portion 22 of the aperture 20 in the needle sheath gripping member 4 of the device 2. The needle sheath 654 has been pushed into the needle sheath gripping portion 24 of the aperture 20, such that the sharp opposing edge portions of the gripping portion 24 grip the outer surface of the needle sheath 654. The syringe 650 has been pulled upwards to remove the needle 652 from the needle sheath 654. The needle sheath 652 may be replaced over the needle 652 and onto the syringe 650 by reversing this procedure.

## Claims

1. A device for removing and replacing a needle sheath of a needle device, the device comprising:
a support;
a needle sheath gripping member having an aperture therein, the aperture comprising:
a needle sheath receiving portion having a first diameter for receiving a portion of the needle sheath therein; and
a needle sheath gripping portion having a second diameter for gripping a portion of the needle sheath, wherein the second diameter is less than the first diameter, the sheath gripping portion comprising an edge for gripping the outer surface of the needle sheath.

2. The device according to claim 1, wherein the support comprises a gripping portion that may be gripped by a user to hold the device and orient the needle sheath gripping portion for removing the sheath from covering the needle of a medical device and/or replacing the sheath over the needle.

3. The device according to either of claims 1 or 2, wherein the support comprises a stand portion.

4. The device according to claims 1 or 2, wherein the support comprises a portion that provides both the gripping portion and the stand portion.

5. The device according to any preceding claim, wherein the support comprises a first support member extending from a first side of the needle sheath gripping member and a second support member extending from a second side of the needle sheath gripping member; preferably wherein the first side is on an opposite side of the needle sheath gripping member to the second side.

6. The device according to any preceding claim, wherein the needle sheath gripping member is generally planar.

7. The device according to any preceding claim, wherein the needle sheath gripping member and the support are formed as a single component.

8. The device according to any preceding claim, wherein the first diameter is from 4 to 11mm and/or wherein the second diameter is from 2 to 9mm.

9. The device according to any preceding claim, wherein the edge of the needle sheath gripping portion is provided with one or more chamfers; preferably wherein the edge of the needle sheath gripping portion of the aperture comprises two opposing chamfer portions for gripping opposing portions of the outer surface of the needle sheath; and/or wherein a chamfer is provided on substantially the entire length of the edge of the needle sheath gripping portion; and/or wherein part or all of the edge of the needle sheath receiving portion of the aperture is provided with a chamfer.

10. The device according to claim 9, wherein the or each chamfer extends at an angle of from 22.5 to 37.5°.

11. The device according to any preceding claim, wherein the portions of the edge of the needle sheath receiving portion of the aperture adjacent or closest to the needle sheath gripping portion are provided with a chamfer; preferably wherein, the entire needle sheath receiving portion of the aperture is provided with a chamfer.

12. The device according to any preceding claim, wherein the aperture comprises a transition portion extending between the needle sheath receiving portion and the needle sheath gripping portion; preferably wherein the portions of the edge of the transition portion of the aperture adjacent or closest to the needle sheath gripping portion are provided with a chamfer.

13. The device according to any preceding claim, wherein the apertures comprises a plurality of needle sheath gripping portions.

14. The device according to any preceding claim, wherein the shape of the aperture is a teardrop shape, generally triangular or diamond shaped.

15. A method for removing a needle sheath from a needle device, the method comprising:
inserting a portion of the needle sheath into a receiving portion of an aperture in a needle sheath gripping member;
moving the needle sheath into a gripping portion of the aperture, whereby a portion of the outer surface of the needle sheath is gripped by opposing edge portions of the gripping portion of the aperture; and
pulling the needle device away from the needle sheath gripping member;
preferably further comprising the steps of:
inserting the needle of the needle device into the needle sheath and reengaging the needle sheath with the needle device;
moving the needle sheath from the gripping portion to the receiving portion of the aperture; and
pulling the needle device away from the needle sheath gripping member.

16. The method according to claim 15, wherein the needle sheath gripping member is supported on a surface.

17. The method according to either of claims 15 or 16, wherein a device according to any of claims 1 to 14 is employed.
